# EUROPEAN PATENT APPLICATION

(11) **EP 2 540 316 A1**
(43) Date of publication of application: **02.01.2013**
(21) Application number: 10846634.3
(22) Date of filing: 03.12.2010
(51) Int. Cl.: A61K 45/06, A23L 1/30, A61K 36/18, A61K 36/48, A61K 36/60, A61K 38/00, A61P 3/04

(54) **COMPOSITION FOR CONTROLLING WEIGHT GAIN AND FOOD PRODUCT CONTAINING THE SAME**

(30) Priority: 25.02.2010 JP 2010040502
(71) Applicant: FUJIFILM Corporation, Tokyo 106-8620 (JP)
(72) Inventor: ODA, Yuriko, Ashigarakami-gun Kanagawa 258-8577 (JP); UEDA, Fumitaka, Ashigarakami-gun Kanagawa 258-8577 (JP)
(74) Representative: HOFFMANN EITLE
(86) International application number: PCT/JP2010/071743
(87) International publication number: WO 2011/104971

(57) **Abstract**

The present invention provides a body weight gain suppressing composition including an α-glucosidase activity inhibitor component, preferably a component derived from at least one plant selected from the group consisting of *Salacia* genus plants, touchi and mulberry, and a lipid synthesis inhibitor component, preferably, at least one selected from the group consisting of soybean peptide, marine animal peptide, and sesame lignin, and also provides a food product including the composition.

## Description

### Technical Field

The present invention relates to a body weight gain suppressing composition and a food product comprising the composition.

### Background Art

Due to body weight gain caused by a high fat food based dietary life associated with westernization of diet, as well as by lack of exercise and the like, obesity is recently becoming a social issue. Obesity carries risks of developing lifestyle-related diseases such as diabetes, hypertension, hyperlipidemia or the like. Thus, suppressing body weight gain has been thought to be important for lowering the incidence of such lifestyle-related diseases.

It has been widely recognized that improvements in lifestyle, including doing an appropriate amount of exercise and adjusting dietary content, is effective for suppressing body weight gain. Nonetheless, with medicine or a food product for suppressing obesity or body weight gain, body weight should be able to be controlled more simply and conveniently. From such a point of view, a variety of components and food products capable of suppressing body weight gain have been proposed. In particular, when compared with synthetic medicine, a naturally-derived component may meet less psychological resistance and be easily accessible to users.

For instance, Japanese Patent Application Laid-Open (JP-A) No. 11-246427 discloses a sugar and lipid metabolism activating agent comprising sesamin or sesamol which is a fraction of defatted sesame, the fraction being soluble in methanol and in 1-butanol, and describes that the sugar and lipid metabolism activating agent contributes to sugar metabolism and/or lipid metabolism activation, and is able to prevent or treat obesity and the like.

JP-A No. 2007-330124 discloses a composition for health food to prevent and/or improve obesity and lifestyle-related diseases, the composition comprising *Hoodia gordonii,* wheat extract, mushroom chitosan, soy pulp (*okara*) and the like.
JP-A No. 2009-179579 discloses a lipase activity inhibitor comprising an extract of *Salacia reticulata* leaf and the like, and describes that the inhibitor is capable of effectively and safely preventing, treating, and remedying obesity and hyperlipidemia by strongly inhibiting pancreatic lipase activities.

However, from the viewpoint of suppressing body weight gain, it cannot be said that any of the compositions disclosed in the above documents is sufficient.

### SUMMARY OF INVENTION

### Technical Problem

Accordingly, an object of the present invention is to provide a body weight gain suppressing composition capable of suppressing increases in body weight more effectively and a food product comprising the composition.

### Solution to Problem

According to a first aspect of the present invention, a body weight gain suppressing composition comprising an α-glucosidase activity inhibitor component and a lipid synthesis inhibitor component is provided.
According to a second aspect of the present invention, the body weight gain suppressing composition of the first aspect, wherein the α-glucosidase activity inhibitor component is derived from at least one plant selected from the group consisting of *Salacia* genus plants, mulberry and touchi, is provided.
According to a third aspect of the present invention, the body weight gain suppressing composition of the first aspect, wherein the α-glucosidase activity inhibitor component is at least one selected from the group consisting of a *Salacia* genus plant extract, a mulberry leaf extract and a touchi extract, is provided.
According to a fourth aspect of the present invention, the body weight gain suppressing composition of any one of the first to third aspects, wherein the lipid synthesis inhibitor component is at least one selected from the group consisting of soybean peptide, marine animal peptide and sesame lignin is provided.
According to a fifth aspect of the present invention, the body weight gain suppressing composition of any one of the first to fourth aspects, wherein the content of the lipid synthesis inhibitor component is from 0.005 times to 200 times of the total mass of the α-glucosidase activity inhibitor component, is provided.
According to a sixth aspect of the present invention, the body weight gain suppressing composition of any one of the first to fifth aspects, wherein the content of the α-glucosidase activity inhibitor component is from 0.0001 mass% to 99 mass% of the total mass of the composition, is provided.
According to a seventh aspect of the present invention, the body weight gain suppressing composition of any one of the first to sixth aspects, wherein the content of the lipid synthesis inhibitor component is from 0.0001 mass% to 99 mass% of the total mass of the composition is provided.
According to a eighth aspect of the present invention, the body weight gain suppressing composition of any one of the first to seventh aspects, wherein the α-glucosidase activity inhibitor component is a *Salacia* genus plant extract, and the lipid synthesis inhibitor component is at least one of marine animal peptide or sesame lignan, is provided.
According to a ninth aspect of the present invention, a food product comprising the body weight gain suppressing composition of any one of the first to eighth aspects is provided.

### Advantageous Effects of Invention

According to the present invention, a body weight gain suppressing composition capable of suppressing increases in body weight more effectively and a food product comprising the composition can be provided.

### DESCRIPTION OF EMBODIMENTS

A body weight gain suppressing composition according to the present invention is a body weight gain suppressing composition comprising an α-glucosidase activity inhibitor component and lipid synthesis inhibitor component.
Because the body weight gain suppressing composition according to the present invention comprises the α-glucosidase activity inhibitor component and lipid synthesis inhibitor component, it is possible to promote breakdown of fat in the body as well as to inhibit fat synthesis, thereby more effectively suppressing increase in body weight. That is, the present invention is based on a finding that the α-glucosidase activity inhibitor component has an effect of promoting breakdown of fat, and finds that, when combined with lipid synthesis inhibitor component, increase in body weight is further markedly suppressed.

As used herein, the term "process" means not only an individual process but also a process in which an expected effect in the process is attained even when the process cannot be clearly distinguished from one or more other processes.
As used herein, a numerical range expressed by using "from A to B" indicates a range including A and B as the minimum value and the maximum value respectively.
When the amount of a component is referred in the present invention, in cases where there are two or more substances corresponding to one component of the composition, unless specified that the amount is an amount of a single substance, the amount of the component means the total amount of the two or more substances.
The present invention is described below.

An α-glucosidase activity inhibitor component in the present invention can be any component as long as it inhibits α-glucosidase present in small intestinal epithelia.
Examples thereof include acarbose, voglibose, miglitol, salacinol, kotalanol, deoxynojirimycin, and acteoside. Examples of the α-glucosidase activity inhibitor component further include natural plant-derived components. Examples of the plant-derived α-glucosidase activity inhibitor component include natural components derived from a plant such as guava, touchi, *Glycyrrhiza,* wheat, *Salacia* genus plants, mulberry, rose flower, *Gymnema, Araliaceae* plants (for example, araliad, *udo,* Japanese angelica tree, and the like; hereinafter the same), *Eucalyptus,* cinnamon bark, loquat, *Apocynum venetum* or the like. One kind of these may be used singly or two or more kinds thereof may be used in combination.

Concrete examples of the natural α-glucosidase activity inhibitor component include Guava leaf polyphenol, a touchi extract, a licorice extract, wheat albumin, a *Salacia* extract (ground product or extract of *Salacia* genus plant(s)), a mulberry leaf extract, a rose flower extract, powdered *Gymnema* leaf, *Araliaceae* plant(s), *Eucalyptus,* cinnamon bark, loquat leaf, *Apocynum venetum* and the like. Among these, preferred is acarbose, voglibose, deoxynojirimycin, Guava leaf polyphenol, ground product or extract of *Salacia* genus plant(s), mulberry leaf extract, powdered *Gymnema* leaf or touchi extract. It is salacinol or kotalanol that is particularly preferred as the α-glucosidase inhibition component.
These may be chemical synthetic products. Yet, it is preferred to be used in the form of ground products or extracts of natural products. In the case of the extract *of Salacia* genus plant(s), a hot water extract or alcohol extract may be used.
One kind of these may be used singly, or two or more kinds thereof may be used in combination.

Of these, a *Salacia* genus plant is a plant of *Celastraceae* family which grows wild mainly in Sri Lanka, India, and Southeastern Asian area. More specifically, one or more plants selected from *Salacia reticulata* (*S. reticulata*), *Salacia oblonga* (*S. oblonga*), *Salacia prinoides* (*S. prinoides*), *Salacia chinensis* (*S. chinensis*) are used. One obtained by grinding such plant(s), and an extract powder obtained by extracting from edible parts such as roots, trunks, leaves, flowers, fruits, and the like are used. One or more parts may be mixed to be used. More preferably, the extract powder obtained by extracting from the root and/or trunk is used.

In cases where the extract powder of *Salacia* genus plant(s) is used, the extract powder of *Salacia* genus plant(s) is a product obtained subjecting the edible part(s) described above to solvent extraction and drying the resulting product. The extraction solvent may be selected from water; alcohol such as methanol or ethanol; or a mixed solvent of water and alcohol(s) or ketone(s) such as acetone. Preferably, water, alcohol, or hydrous alcohol is used. More preferably, hot water or ethanol or hydrous ethanol is used as the extraction solvent. The alcohol concentration of the above-mentioned hydrous alcohol may be, for example, from 30 to 90%, preferably from 40 to 70%. A method of drying includes spray drying, freeze drying and the like, but is not limited thereto.

It is preferred that an α-glucosidase inhibition component in the present invention have a sucrase 50% inhibitory concentration (IC₅₀ value) of from 0.0001 µg/ml to 800 µg/ml. The sucrase 50% inhibitory concentration is more preferably from 0.001 µg/ml to 600 µg/ml, and still more preferably from 0.001 µg/ml to 450 µg/ml. The sucrase 50% inhibitory concentration (IC₅₀ value) can be measured by the method described in paragraphs [0009] to [0012] in JP-A No. 2009-249315.

A lipid synthesis inhibitor component is not particularly restricted as long as it inhibits activity of an enzyme system involved in the fat synthesis pathway. Such a lipid synthesis inhibitor component includes soybean peptide, marine animal peptide (collagen peptide), sesame lignan, ginger extract, and the like.
The marine animal peptide here includes collagen obtained by extracting collagenous tissues of fish (saltwater fish). Examples of a raw material of the collagen include skin of tuna (yellowfin tuna), shark, cod, flounder (or *hirame*), righteye flounder (or *karei*), sea bream, tilapia, salmon and the like.

A fat synthesis inhibiting activity can be confirmed by a known evaluation system. For instance, any substance can be used for the present invention as long as it has, when taken orally, an action of reducing the expression level of mRNA of sterol regulatory element binding protein 1 (SREBP-1), which is a protein playing a role in regulating the lipid synthesis in the liver.

A body weight gain suppressing composition of the present invention may be in the form of any of liquid, solid, powder, and gel. It may be also in the form of solution, tablet, hard capsule, soft capsule, granule or the like.

The content of the α-glucosidase activity inhibitor component in the body weight gain suppressing composition varies depending on a dosage form or administration mode of the body weight gain suppressing composition. It can be any amount as long as it is an amount effective for activities of breakdown of lipid. For instance, when the body weight gain suppressing composition is in the form of solution, the content of the α-glucosidase activity inhibitor component can be from 99 mass% to 0.0001 mass% of the total mass of the composition, preferably from 90 mass% to 0.0005 mass%; when the body weight gain suppressing composition is in the form of solid, the content of the α-glucosidase activity inhibitor component can be from 95 mass% to 0.0001 mass%, and preferably from 90 mass% to 0.0001 mass%; of the total mass of the composition, but the content of the α-glucosidase activity inhibitor component is not particularly restricted. The dose varies depending on the dosage form and the like. In general, the dose can be from 0.5 mg to 1500 mg once a day, and preferably from 2 mg to 800 mg once a day, as the α-glucosidase activity inhibitor component, but is not particularly restricted.

The content of the lipid synthesis inhibitor component in the body weight gain suppressing composition varies depending on a dosage form or administration mode of the body weight gain suppressing composition. It is not particularly restricted as long as it is an amount effective for activities of inhibiting lipid synthesis. For instance, when the body weight gain suppressing composition is in the form of solution, the content of the lipid synthesis inhibitor component can be from 99 mass% to 0.0001 mass%, and preferably from 90 mass% to 0.0005 mass%, of the total mass of the composition; when the body weight gain suppressing composition is in the form of solid, the content of the lipid synthesis inhibitor component can be from 95 mass% to 0.0001 mass%, and preferably from 90 mass% to 0.0001 mass%, of the total mass of the composition; but the content of the lipid synthesis inhibitor component is not particularly restricted. The dose varies depending on the dosage form and the like. In general, the dose can be from 0.01 mg to 3000 mg once a day, and preferably from 1 mg to 1500 mg once a day, as the fat synthesis inhibitor component, but is not particularly restricted.

A ratio between the amount of the α-glucosidase activity inhibitor component and that of the lipid synthesis inhibitor component can be any as long as each component is within the above content range. From the viewpoint of ease of intake, the mass of the lipid synthesis inhibitor component can be preferably from 0.005 times to 200 times, and more preferably 0.01 times to 100 times, of the total mass of α-glucosidase activity inhibitor component.

The body weight gain suppressing composition of the present invention may contain a pharmaceutically acceptable carrier or any other well known additional component according to its dosage form or administration mode. In cases where it is in the form of solution, examples of a preferred carrier used include an aqueous medium such as water or the like. Examples of a preferred additional component which can be used to form into a solid includea diluent such as crystalline cellulose or magnesium stearate and a bulking agent such as corn starch or alginic acid. Examples of a coating agent which can be used for tablets, capsules, and granules include shellac, sugar, film coating base material, and YEAST WRAP.

In the present invention, in order to improve time-dependent discoloration of *Salacia* genus plant extract powder, it is preferred that calcium carbonate or silicon dioxide is included in an amount of not less than 1% of the mass of the tablet or hard capsule. Furthermore, a low moisture absorption raw material or moisture absorbent utilizable as a food product or food product additive can be used. Preferably, as the low moisture absorption raw materials, cellulose, crystalline cellulose, powdered cellulose, microcrystalline cellulose, lactose, oligosaccharide, sugar alcohol, trehalose, magnesium stearate, calcium stearate or the like may be used. As the moisture absorbent, silicates, magnesium carbonate, ferrocyanide, polysaccharide or the like may be used. More preferably, as the low moisture absorption raw material, crystalline cellulose, microcrystalline cellulose or lactose is used. Examples of a compound necessary for forming into a powder, solid formulation or liquid formulation includes erythritol, maltitol, hydroxypropylcellulose, kaolin, talc and the like.

As an administration mode of the body weight gain suppressing composition, oral administration is preferred. Yet, parenteral administration, for example rectal administration or sublingual administration may be employed.

A body weight gain suppressing composition of the present invention can suppress increase in body weight simply and conveniently as well as effectively, and hence it can be preferably used as a food product. That is, the present invention also provides the food product comprising the above body weight gain suppressing composition.
The food product according to the present invention can be any food product as long as it includes the body weight gain suppressing composition. Matters concerning the body weight gain suppressing composition are applicable as they are to the food product of the present invention.
In the food product recited in the present invention, the content of the body weight gain suppressing composition may be any, as long as it is in a range where effects of the body weight gain suppressing composition are attained. For instance, the content may be not less than 0.001 mass%.

### EXAMPLES

By way of examples, the present invention is described in detail below. However, the present invention is by no means limited thereto. Unless otherwise noted, "part" is based on mass.

### Example 1

The fat breakdown promotion by an α-glucosidase activity inhibitor component was checked as described below.

Eight-week-old male SD rats were randomly divided into groups of 7 rats. The rats were allowed to freely take high fat food (HFD32, manufactured by CLEA Japan, Inc.), and were also given a sample in an amount described in Table 1 by a gastric tube, for 30 days. The composition of each sample is as follows. The control group was administered with water for injection.

As active components of the samples described in Table 1, the following components were used. These components were each individually dissolved in water for injection and the resulting solution was administrated as an aqueous solution having a concentration of 10 mg/ml.
Sample B has a *Salacia* genus plant extract as the active component. As the *Salacia* genus plant extract, a *Salacia* extract powder prepared by grinding the root and trunk parts of *Salacia reticulata* (*S. reticulata*) and *Salacia oblong* (*S. oblonga*) and subjecting the resultant to a process of a hot water extraction at 98°C followed by spray drying the resulting liquid, was used.
Sample C has a mulberry leaf extract as the active component. The mulberry leaf extract used was prepared by adding 300 g of mulberry leaf dry powder with 1 liter of 25% (v/v) ethanol to perform extraction, and removing solvents from the resulting filtrate under reduced pressure followed by drying.
Sample D has a touchi extract as the active component. As the touchi extract, the powderized product obtained by freeze drying commercially available touchi was used.
With respect to the samples containing any of these three kinds of active components, the sucrase IC₅₀ was examined. It was confirmed that all of them had a sucrase IC₅₀ of not more than 450 µg/ml.
After a 30-day feeding period, blood was collected and blood serum was then separated in accordance with a conventional method to measure the amount of neutral fat in the blood serum. The results are shown in Table 1.

**Table 1**

| Sample | Component contained | Amount of active component | Amount of serum neutral fat [mg/dL] |
|---|---|---|---|
| A | - | | 170.3 ± 5.6 |
| B | *Salacia* genus plant extract | 20 mg/kg/day | 135.9 ± 4.4** |
| C | Mulberry leaf extract | 500 mg/kg/day | 152.4 ± 2.9* |
| D | Touchi extract | 20 mg/kg/day | 146.0 ± 9.1** |

| | | | |
|---|---|---|---|
| * P < 0.05 ** P < 0.01 | | | |

As shown in Table 1, in the groups administered with samples B to D exhibited, as compared with the non-administration group, significantly enhanced breakdown of lipid. It is therefore found that all of the *Salacia* genus plant extract, mulberry leaf extract, and touchi extract promote the breakdown of lipid.
All of the *Salacia* genus plant extract, the mulberry leaf extract, and the touchi extract significantly decreased neutral fat in the blood serum, when compared with the case where the rats were given with high fat food alone. Using the *Salacia* genus plant extract, the mulberry leaf extract, and the touchi extract, Example 2 was carried out.

### Example 2

Next, using samples in which any of the *Salacia* genus plant extract, the mulberry leaf extract, and the touchi extract, all which were used in Example 1, and the lipid synthesis inhibitor component described in Table 2, suppression of increase in body weight was evaluated in the following manner.
Eight-week-old male SD rats which had been fed with high fat food (HFD32, manufactured by CLEA Japan, Inc.) for 30 days were divided into groups of 7 rats. The body weight of each rat was measured. And then, while feeding with the high fat food was continued, a sample with the composition described in Table 2 was given to the rat by a gastric tube for 30 days. The control group was administered with water for injection alone.
After the 30-day administration, the body weight was measured. The results are shown in Table 2. The amount of each component in Table 2 is a dose per day.

**Table 2**

| Sample | α-glucosidase activity inhibitor component | Lipid synthesis inhibitor component | Ratio of increase in body weight (%) |
|---|---|---|---|
| A | - | - | 143 ± 2 |
| B | *Salacia* genus plant extract (20 mg/kg) | - | 128 ± 1** |
| C | Touchi extract (20 mg/kg) | - | 135±5 |
| D | Mulberry leaf extract (500 mg/kg) | - | 141 ± 1 |
| E | - | Soybean peptide (200 mg/kg) | 135 ± 1* |
| F | - | Marine animal peptide (200 mg/kg) | 132 ± 3* |
| G | - | Sesame lignan (1 mg/kg) | 126 ± 3** |
| H | *Salacia* genus plant extract (20 mg/kg) | Soybean peptide (200 mg/kg) | 120 ± 1** |
| I | *Salacia* genus plant extract (20 mg/kg) | Marine animal peptide (200 mg/kg) | 103 ± 4** |
| J | *Salacia* genus plant extract (20 mg/kg) | Sesame lignan (1 mg/kg) | 97 ± 6** |
| K | Touchi extract (20 mg/kg) | Soybean peptide (200 mg/kg) | 129 ± 7* |
| L | Touchi extract (20 mg/kg) | Marine animal peptide (200 mg/kg) | 125 ± 2** |
| M | Touchi extract (20 mg/kg) | Sesame lignan (1 mg/kg) | 122 ± 2** |
| K | Mulberry leaf extract (500 mg/kg) | Soybean peptide (200 mg/kg) | 130 ± 6* |
| N | Mulberry leaf extract (500 mg/kg) | Marine animal peptide (200 mg/kg) | 127 ± 7* |
| O | Mulberry leaf extract (500 mg/kg) | Sesame lignan (1 mg/kg) | 122 ±1** |

| | | | |
|---|---|---|---|
| * P < 0.05 ** P < 0.01 | | | |

As shown in Table 2, when compared with the α-glucosidase activity inhibitor component alone (Samples B to D) or the lipid synthesis inhibitor component alone (Samples E to G), combination of each α-glucosidase activity inhibitor component with each lipid synthesis inhibitor component was found to markedly increase the effect of suppressing increase in body weight (Samples H to O).
In particular, combining *Salacia* genus plant extract used as the α-glucosidase activity inhibitor component with sesame lignan or marine animal peptide as the lipid synthesis inhibitor component was found to result in a particularly profound body weight gain suppressing effect (Samples H to J).

Hence, according to the present invention, suppression of increase in body weight can be more effectively achieved.

The disclosure of Japanese Patent Application No. 2010-040502, filed on February 25, 2010, is incorporated herein by reference in its entirety.
All of the documents, patent applications and technical standards cited in this specification are herein incorporated by reference to the same extent as if each individual document, patent application and technical standard were specifically and individually indicated to be incorporated by reference.

## Claims

1. A body weight gain suppressing composition comprising an α-glucosidase activity inhibitor component and a lipid synthesis inhibitor component.

2. The body weight gain suppressing composition according to claim 1, wherein the α-glucosidase activity inhibitor component is derived from at least one plant selected from the group consisting of *Salacia* genus plants, mulberry and touchi.

3. The body weight gain suppressing composition according to claim 1, wherein the α-glucosidase activity inhibitor component is at least one selected from the group consisting of a *Salacia* genus plant extract, a mulberry leaf extract and a touchi extract.

4. The body weight gain suppressing composition according to any one of claims 1 to 3, wherein the lipid synthesis inhibitor component is at least one selected from the group consisting of soybean peptide, marine animal peptide and sesame lignin.

5. The body weight gain suppressing composition according to any one of claims 1 to 4, wherein the content of the lipid synthesis inhibitor component is from 0.005 times to 200 times the total mass of the α-glucosidase activity inhibitor component.

6. The body weight gain suppressing composition according to any one of claims 1 to 5, wherein the content of the α-glucosidase activity inhibitor component is from 0.0001 mass% to 99 mass% of the total mass of the composition.

7. The body weight gain suppressing composition according to any one of claims 1 to 6, wherein the content of the lipid synthesis inhibitor component is from 0.0001 mass% to 99 mass% of the total mass of the composition.

8. The body weight gain suppressing composition according to any one of claims 1 to 7, wherein the α-glucosidase activity inhibitor component is a *Salacia* genus plant extract, and the lipid synthesis inhibitor component is at least one of marine animal peptide or sesame lignan.

9. A food product comprising the body weight gain suppressing composition according to any one of claims 1 to 8.
